# EUROPEAN PATENT SPECIFICATION

(11) **EP 1 644 015 B1**
(45) Date of publication and mention of the grant of the patent: **27.02.2008**
(21) Application number: 04738046.4
(22) Date of filing: 28.06.2004
(51) Int. Cl.: A61K 36/57, A61P 15/12

(54) **PLANT EXTRACTION METHOD AND EXTRACT**
PFLANZENEXTRAKTIONSVERFAHREN UND EXTRAKT
PROCEDE D'EXTRACTION VEGETALE ET EXTRAIT

(30) Priority: 01.07.2003 CH 116203
(43) Date of publication of application: 12.04.2006
(73) Proprietor: Max Zeller Söhne AG, 8590 Romanshorn (CH)
(72) Inventor: BRATTSTRÖM, Axel, 78476 Allensbach (DE)
(74) Representative: Ritscher, Thomas
(86) International application number: PCT/CH2004/000405
(87) International publication number: WO 2005/002609

(56) References cited:
- EP-A- 0 324 197
- WO-A-01/05415
- WO-A-02/30436
- WO-A-02/067962
- WO-A-03/000074
- WO-A-03/037096
- US-A- 4 985 247
- US-A1- 2003 054 050
- US-B1- 6 267 994
- LISKE E: "Therapeutic Efficacy and Safety of Cimicifuga racemosa for Gynecologic Disorders" ADVANCES IN THERAPY, HEALTH COMMUNICATIONS, METUCHEN, NJ, US, vol. 15, no. 1, 1998, pages 45-53, XP001068814 ISSN: 0741-238X
- ZHENG Q Y ET AL: "Chapter 33: CIMIPURE (CIMICIFUGA RACEMOSA): A STANDARDIZED BLACK COHOSH EXTRACT WITH NOVEL TRITERPENE GLYCOSIDE FOR MENOPAUSAL WOMEN" PHYTOCHEMICALS AND PHYTOPHARMACEUTICALS, XX, XX, 2000, pages 360-370, XP001069523
- PATENT ABSTRACTS OF JAPAN vol. 1997, no. 06, 30 June 1997 (1997-06-30) & JP 9 030977 A (NANBA TSUNEO; KADOTA SHIGETOSHI; KAKEN PHARMACEUT CO LTD), 4 February 1997 (1997-02-04)
- DATABASE WPI Section Ch, Week 198411 Derwent Publications Ltd., London, GB; Class B04, AN 1984-065078 XP002296802 & JP 59 020298 A (SUNTORY LTD) 1 February 1984 (1984-02-01)
- BORRELLI F ET AL: "Cimicifuga racemosa: A systematic review of its clinical efficacy" EUROPEAN JOURNAL OF CLINICAL PHARMACOLOGY, SPRINGER VERLAG, DE, vol. 58, no. 4, July 2002 (2002-07), pages 235-241, XP002237716 ISSN: 0031-6970

## Description

The present invention generally concerns the art of producing extracts from medicinal plants and, specifically, production of extracts from medicinal plants which contain a substantial portion, at least, of triterpene glycosides and/or triterpene saponines as active ingredients.

Important examples of this type of medicinal plants are the *Cimicifuga species,* e.g. *Cimicifuga racemosa* (also termed *Actaea racemosa* L; black cohosh; cohosh bugbane; snakeroot; bugbane) since extracts from various parts of this plant species are known to be effective as analgesics for treatment of pain in muscles and joints, as well as to treat neuralgic diseases rheumatic arthritis, menstrual discomfort, and post-natal pains.

For example, such extracts have been disclosed in WO 99/47149 as an organo-selective estrogen-type medicament for selective treatment and/or prophylaxis of cardiovascular diseases and climacteric discomfort. However, no uterotropic effects have been observed.

Further, US 6,267,994 discloses use of an extract from *Cimicifuga racemosa*, notably for anti-tumour therapy in compositions that do not exhibit the toxicity of high doses of anti-estrogenic compounds.

Finally, WO 01/05415 discloses a pharmaceutical composition containing components of *Cimicifuga racemosa* extract or derivatives thereof that exhibit an estrogen-type effect.

Conventional methods of producing such extracts are based upon treatment of plant material with an aqueous alcanolic extracting agent so as to obtain a raw or primary extract which after an optional treatment for removal of fines, e.g. by sedimentation or filtration, contains the extracting agent and the plant constituents that are soluble in the extracting agent.

Typically, the primary extract is then concentrated by partial evaporation of the extracting agent so as to remove its more volatile components, generally the alcanolic constituents plus some water, and to form what is called a concentrated extract, typically containing 5 - 50 % by volume, of residual solvent, i.e. water. Upon further removal of solvent, a solid, pasty, or liquid material is obtained that is substantially free of the solvent used for extraction of the plant material. This product (also termed "extract") can then be used as such or processed to produce specific application forms, e.g. pills, lotions, solutions, powders etc., optionally adding whatever adjuvants, additives, coating components and the like are needed for the final medicinal product.

Research by applicant leading to the present invention has shown that conventional production methods of extracting medicinal plants that contain a substantial portion, at least, of triterpene glycosides and/or triterpene saponines tend to suffer from a loss of active ingredients and/or yield an inhomogeneous product, and that this is due to unintended precipitation upon solvent removal. *Cimicifuga species* are an important example of such medicinal plants but not the only one.

Active ingredients of *Cimicifuga racemosa* include triterpene glycoside of the cycloartan-type of which at least 20 species are known. A main ingredient is acteine, 23-epi-26-deoxyactein, 27-deoxyactin and cimiracemoside A. Actein is a xyloside of acetyl acteol with a 16,23:23,26:24,25-triepoxy side chain. 27-Deoxyactine is used as a standard for quantitative determination of the compounds just mentioned. A further known group of active ingredients are aromatic acids such as ferulic acid, hesperatinic acid and acyl caffeinic acid. Further, the following esters of hydroxy cinnamic acid have been isolated from aqueous ethanolic extracts of *Cimicifuga racemosa:* fucinolic acid and the cimicifugaic acids A, B, E und F. Flavonoides, biochanine A, formononetine and camphorol have also been reported. Other ingredients are tannins, resins and fatty acids (oleic acid, linolic acid, linolenic acid and palmitinic acid.

With such a large number of constituents it is easily understandable that extraction conditions will have a substantial impact upon the composition of the resulting extracts and that any unintended change of the proportional makeup is undesirable.

Accordingly, it is a first object of the present invention to provide for a new method of extracting such medicinal plants that contain a substantial portion, at least, of triterpene glycosides and/or triterpene saponines, which method is capable of preventing such loss of active ingredients upon solvent removal so as to yield novel extracts of improved composition.

Another object of the invention is an extract of *Cimicifuga species* such as *Cimicifuga racemosa* having a composition that differs from prior art extracts.

A further object of the invention is to provide for medicaments comprising such extracts, as well as for medical treatment of diseases in animal patients including humans using such extracts.

WO 03/000074 and EP 1 407 679 disclose a process for producing a liquid plant extract, e.g. from *Hydrangea dulcis folium* by concentrating a liquid extract in the presence of a plant powder made, for example, from *Hydrangea dulcis folium.*

WO 03/037096 discloses the preparation of extracts from botanic materials wherein at least one acid and an antioxidant, e.g. citric acid and ascorbic acid is added prior to concentration.

According to WO 02/67962 gelatin is added in the preparation and concentration *of Anemone raddeana* extract.

WO 02/30436 discloses extraction of *Phyllanthus* and concentrating the extract after addition of tragacanth gum and maltodextrin.

EP 0 324 197 suggests to use to prepare ginko extract by concentration in the presence of kieselguhr.

Accordingly, the art can be summarized in that various additives, soluble or insoluble in the extraction liquid, have been suggested an used to improve the concentration process when preparing plant extracts.

It has been found, according to the invention, that novel extracts including, but not restricted to, those obtainable from various parts of the plant species *Cimicifuga* differing in composition from prior art extracts can be obtained if such unintended precipitation is prevented, and that such prevention can be effected if removal of the extracting solvent is effected in the presence of a polyvinylpyrrolidon (PVP) as a solution mediator.

Now, according to a first general embodiment the invention provides for a method of producing a vegetal plant extract by the steps of:
(A) treating a material (e.g. leaves, roots, stems, branches, seeds) of a medical plant, preferably a *Cimicifuga species,* with an extraction agent to obtain a primary extract;
(B) separating said primary extract from said solid material; and
(C) concentrating said primary extract for removing a portion, at least, of said extraction agent for obtaining an essentially homogeneous concentrate;
wherein said step (C) of concentrating said primary extract is effected in the presence of an effective amount of a PVP as a pharmacologically acceptable and physiologically inert solution mediator so as to substantially avoid loss of constituents of said primary extract.

According to a preferred embodiment of the inventive method, the product obtained in step (C) is subjected to a further treatment for substantially removing all of the extraction agent, preferably by spray drying, to obtain an essentially dry product.

Medical plants other than *Cimicifuga species* for use in preparing extracts according to the inventive method include *Hedera, Liquorice, Symphytum, Centella asoatica*, *Ginseng and Solidago.* Generally, all medical plant extracts can benefit from the inventive teaching of adding a solution mediator prior to concentration provided that the PVC does not form a precipitate with constituents of the extract, e.g. tannic acids.

Generally, the term "extract" as used herein without further specification is intended to generally refer to any form of the product of extraction minus the extracting agent and regardless of the physical form (i.e. viscous, pasty or solid).

PVP as solution mediators for use in the invention are macromolecular substances or polymers known to be admissible for use in medicaments and to be physiologically inert as well as soluble in the extraction agent and in water. The term "soluble" as used herein does not necessarily indicate complete solubility but a partial solubility in the extraction agent as well, as is generally apparent from the fact that the PVP is capable of being dissolved in the extraction agent at temperatures between 0°C and 100 °C at ambient pressure so as to form a "normal" (clear) or "colloidal" (turbid) solution, also termed a colloidal or molecular dispersion. Preferably, the PVP should be capable of forming an aqueous solution which, at room temperature, contains at least about 30% by weight of PVP.

The molecular mass of such a PVP generally is in the range of from about 1'000 to about 1'300'000 Dalton. A minimum molecular mass of suitable PVP of about 5'000 Dalton is preferred for many purposes of the invention.

PVPs are admitted under current food and drug regulations for pharmaceutical or food use and are available commercially from various sources.

As has been shown in many studies, PVP when ingested by humans has no negative effects and is not digested or metabolized. When taken orally, the predominant portion thereof is excreted within 11 hours and no PVP enrichment has been observed.

Addition of a PVP not only prevents agglomeration and subsequent precipitation of less soluble components of the primary extract upon concentration in the manner of a protective colloid but additionally is able to retain the entire content of the plant extract upon complete removal of the extraction agent without loss of extracted plant components.

An effective amount of PVP generally is in the range of from about 5 % by weight to about 50 % by weight, based upon the weight of the final extract. A range of from about 15 % by weight to about 35 % by weight, is preferred for many purposes.

Preferably, the extraction agent is a polar substance, such as a normally liquid alcohol, e.g. methanol, ethanol, propanol, butanol as well as mixtures of such C₁₋₄ alcohols with each other and/or with water. Mixtures of from about 40 to about 80 %, by weight, of such alcanol, preferably ethanol, with about 60 to about 20 %, by weight, of water are preferred for many purposes. Whenever the qualificator "about" is used herein, it is intended to include a variation of ± 30%, preferably ± 10%, from the value associated with this qualificator.

Preferably, the PVP is added to the primary extract rather than to the extraction agent. The extraction procedure may be carried out in conventional apparatus. In any case, the solution mediator is added prior to any significant removal of extraction agent. Such removal or concentration can be effected in a known manner, e.g. by distillation at an elevated temperature and/or reduced pressure taking care to avoid conditions that could affect the components of the extract. Concentration may be effected at constant or varying conditions of temperature and/or pressure.

A preferred embodiment of the inventive process is carried out by extraction of the dry plant material with an ethanol-water mixture containing about 50 - 60 %, by volume, of ethanol und about 50 - 40 %, by volume, of water. Upon concentration, the alcanolic constituents plus any water of an azeotropic mixture is removed until a solids concentration of about 25 - 50 %, by weight, is reached. The concentrated extract obtained may then by processed in any conventional manner, e.g. by spray-drying to form a dry product. During such a final concentration step, e.g. upon spray-drying the solution mediator PVP, additionally acts as a spray-drying adjuvant. Generally, the PVP has the effect that a homogeneous consistence of the extract is maintained in all stages of the concentration process.

Extracts of *Cimicifuga racemosa* obtained as disclosed herein can be used according to the invention for production of a medicament suitable for treating climacteric and post menopausal distress. Such preparation can be effected in conventional manner except that the extract used has been obtained in the presence of PVP according to the invention.

Such a medicament is suitable as a substitute or complement of a hormone replacement therapy, and is capable of alleviating climacteric and menopausal symptoms, therefore it can generally be applied for treating climacteric and post menopausal distress in a female human patient.

A medicament according to the invention normally contains the novel extract as an at least dominant portion in combination with other conventional and well-known components of medicaments which, in turn, may but need not be pharmaceutically active. On the other hand, a medicament according to the invention may substantially consist of the novel extract, e.g. in a liquid or solid diluent.

It has been found according to the invention that the physiological effectiveness of the novel extract can be determined by a relatively simple measuring method, namely by measuring the pulse frequency of an epithelia sample taken from the oviduct as known in the art of determining the effectiveness of hormone treatment (e.g. Mahmood, T. et al. "The effect of ovarian steroids on epithelial ciliary beat frequency in human fallopian tube" (1998), Human Reproduction, 13(11); 2991 - 2994)

The extract according to the invention can be used to prepare medicaments with an effect similar to that of progesterone effective for treatment of climacteric or post-mensal discomfort in female human patients. To this end, the extract according to the invention can be processed, in a manner known per se from standard pharmacological practice to obtain conventional application forms and applied for medical treatment in the dosages known for use of conventional extracts of *Cimicifuga racemosa,* e.g. in the broad range of from about 0.1 to about 100 mg/kg of body weight. Clinical tests in treating female human patients with menopausal syndrome (cf. example 5 below) gave favorable results when used at a dosage of 6.5 mg and 13 mg/day.

The invention will now be explained in more detail with reference to the enclosed drawing in which:
Figure 1 is a diagram of an LC-analysis of a conventionally prepared extract of *Cimicifuga racemosa*;
Figure 2 is a diagram of an LC-analysis obtained under the same general conditions as in Fig. 1, however with an extract of *Cimicifuga racemosa* obtained according to the invention by concentration in the presence of a solution mediator; and
Figure 3 is a diagram showing results of clinical testing.

In the following examples given by way of illustration not limitation, indications in percent or parts are based upon the weight unless specifically mentioned otherwise.

### Example 1

### Preparation of the extract

Dry root material of *Cimicifuga racemosa (rhizome)* were processed by milling to obtain a powder and charges of material from different harvests were combined for homogeneity.

Extraction was performed on the basis of a ratio of 2'500 parts extraction agent per 500 parts of *Cimicifuga racemosa*. The extraction agent, in turn, was prepared by mixing 1'330 parts of ethanol (96%, by volume) and 1' 170 parts of de-ionized water.

Water and ethanol of ambient temperature were mixed in a container provided with a mechanical stirrer and a withdrawal port at the bottom of the container. Then, the *Cimicifuga racemosa* powder was added with continued stirring, and stirring was continued for 300 minutes. Then, the stirrer was stopped and the solids allowed to settle which normally takes about 180 minutes and, in any case, is continued until a clear supernatant has been formed above the sediment.

The clear supernatant is removed by means of a pump and collected in a separate container. The sediment is fed into a filter press to recover additional extraction liquid which is combined with the supernatant and the resulting mixture is fed through a filter into a mixing container provided with a stirrer and a weighing device. After starting of the poly(vinylpyrrolidone) (termed PVP herein, a commercial product sold under the trade name Plasdone® KW 29-32) is added in an amount of 33 parts per 100 parts of extraction liquid and stirring is continued until the PVP has dissolved and a clear brown liquid is obtained as a first product.

This product is fed into a standard-type two-stage concentrator plant type KV 102 EX produced and sold by Unipektin AG, Eschenz, Switzerland, in which the extraction liquid including the PVP is concentrated by evaporation of the extraction agent to yield a dark brown concentrate having a concentration of at least about 9% solids which is then further concentrated to form a spissum extract having a solids content between about 18 and 26 %. Then, the concentrate is fed into a conventional spray-dryer to produce a dry extract in the form of a powder with a humidity content of not more than about 5% by weight.

Both the concentrated liquid spissum extract as well as the dry pulverulent extract can be standardized as required and used as or in medicaments according to the invention, for example by producing tablets containing 5 - 50 mg extract and the usual solid diluents, additives, and adjuvants for production of tablets having a final weight of about 100 - 200 mg.

### Example 2

Various substances were tested for their effectiveness as solution mediator or protective colloid during concentration. To this end, the criterion of "rimming" was used, i.e. the formation of a distinct "rim" (solidifying deposit at the periphery of the test liquid in a generally round container).

Such rimming normally occurs when concentrating a solution that contains an amorphous or resinous solid and indicates commencement of solidification upon concentration by evaporation of the solvent.

Research leading to the invention has shown that concentration of a solution of plant extract, notably of *Cimicifuga racemosa*, in the extraction liquid by evaporation leads to formation of a "rim" or "border" upon progressing increase of concentration of the solution, and that such rim tends to contain a substantial portion of the active ingredients of the extract. The result of rimming is that rim solids tend to be discarded because a homogeneous product is to be achieved. As a consequence, final extracts obtained by conventional evaporation do not any longer have the same proportional composition as the primary extracts prior to concentration.

On the other hand, it has been found according to the invention that rimming can be prevented or minimized to insignificance if the solution contains PVP as a solution mediator herein, and at a concentration which is sufficient to avoid "rimming".

Thus, the effectiveness of a PVP as a solution mediator for use according to the invention can be determined by a few and simple experiments by observing the amount of rimming and its dependence upon the relative amount of PVP for a given concentration process.

In such experiments, PVP also known as Povidone, is found to substantially preclude rimming when concentrating extracts of C*imicifuga racemosa* and, therefore, is the solution mediator according to the invention for processing extracts of *Cimicifuga racemosa* by evaporation of the extraction solvent. A PVP preferred for many purposes of the invention is obtainable commercially under the trademark Plasdone® K-29/32. This PVP has a molecular mass of about 58'000 Dalton units and a typical viscosity 2,5 mPa·s when measured at standard conditions. This type of PVP highly soluble in water as well as in a lower alcanol, methylene chloride, chloroform and other conventional organic solvents.

Another advantage of PVP is that it is an advantageous additive for processing of solid extracts, such as of *Cimicifuga racemosa,* because - since it can be allowed to remain in the final extract products because of its physiological inactivity - it will facilitate shaping methods of the type used in the production of pharmaceutical tablets. Test with PVP indicate that it can be effectively used in an amount of about 5 to about 50 % by weight, based upon the weight of the final extract or product. A range of from about 15 to about 35 % on the same basis has been found to be particularly effective.

Other substances tested but found to be less effective for extracts of *Cimicifuga racemosa* were hydroxyethyl cellulose (optionally in mixture with lecithin), maltodexrin, hydroxypropyl cellulose, hydroxypropylmethyl cellulose, fructose, tartaric acid (optionally in the form of a salt, e.g. sodium salt).

### Example 3

### Analysis of Extract

The dry extract obtained according to example 1 is a fine lightly brown powder having a characteristic smell. It contains various triterpene glycosides as well as organic acids.

Analytical results of three batches prepared according to example 1 gave the following triterpene glycoside values: 8.34%, 8.84% und 10.04% or an average of 9.07% corresponding to at least 6% if calculated as 27-deoxy acetein, the substance conventionally used for standardization of extracts of *Cimicifuga racemosa*.

These values indicate a most desirable high concentration, notably when considering the fact that the final product contains about 25% of PVP. Conventional extracts of *Cimicifuga racemosa* have a substantially lower content of triterpene glycosides and appear to be less effective at equivalent dosages. The higher and apparently distinctive content of triterpene glycosides in an extract according to the invention is not due to an enrichment procedure but merely upon application of the method according to the invention providing for a recovery of active plant ingredients without loss.

In vitro testing of *Cimicifuga racemosa* extract obtained according to the invention indicates binding to distinguishable somatic receptors as well as to receptors in the central nervous system so as to make such extracts a valuable candidate for alleviating climacteric and menopausal symptoms.

### Example 4

### Comparative Analyses

The operation of example 1 was repeated, however once according to prior art, i.e. without use of a solution mediator during concentration (Sample A), and once with addition of PVP as described in example 1 (Sample B).

For analysis, 0.6 g of each sample were extracted with 25 ml during 15 min in an ultrasonic mixer. An aliquot each of test solution A obtained from sample A, and of test solution B obtained from sample B was transferred by means of a syringe filter into a HPLC vial, and 20 µl were injected into the HPLC apparatus. The column used was a reversed phase RP C-18, 3.0 x 120 mm, 3 µm. The mobile phase was a gradient methanol-water with acetonitrile at a flow rate of 1ml/min. Detection was effected by a ELSD device for detection by evaporation light scattering at an evaporation temperature of 50°C and an evaporation pressure of 3.5 bar. The samples were quantified externally against a 27-deoxyactein standard to determine the triterpene glycosides (TTG).

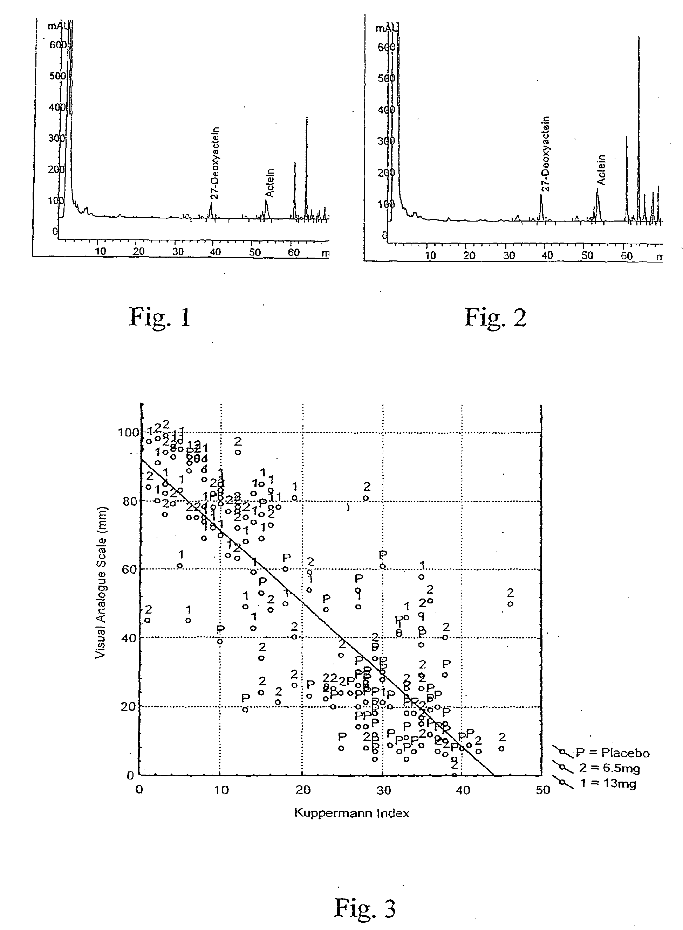

The results are given in Figure 1 for Sample A (prior art extraction method) and in Fig. 2 for sample B (according to the invention; concentration of extract in the presence of solution mediator). As is apparent from a comparison of Fig. 1 and 2, the inventive method provides an extract having a clearly different proportional composition believed to be causal for the advantageous results of clinical testing.

### Example 5

### Clinical Testing

The objective of clinical testing was to evaluate the efficacy and safety of *Cimicifuga racemosa* in the form of tablets prepared from the extract obtained according to example 1 in the treatment of patients with menopausal syndrome.

The test was designed as a prospective, randomised, double-blind, placebo-controlled, parallel group comparison.

Test setting was multicentre in four outpatient general medicine clinics. 180 patients as subjects were randomised and evaluated for the intention-to-treat analysis (60 patients in three groups: placebo, *Cimicifuga racemosa* extract 6.5mg, *Cimicifuga racemosa* extract 13mg). Established diagnostic criteria for menopausal syndrome and validated assessment scales were used.

Interventions: *Cimicifuga racemosa* extract tablets, 13mg; OR 6.5mg ; OR matching placebo tablets once-daily for 12 consecutive weeks.

Main outcome measures: All variables were prospectively sought in a Protocol. Statistical analysis was on an intention-to-treat basis. The main efficacy variable was change from baseline to endpoint (end of Week 12) in menopausal symptoms using the Kuppermann Index (K Index) ( cf. Kuppermann H.S., "Comparative clinical evaluation of estrogen preparations by the menopausal and amenorrhoeal indices." J Clin Endocr 1953; 13: 88). The secondary efficacy variables were: (a) patients' self-assessment of Quality of Life; and (b) Responder Rates. Safety was evaluated by adverse event and clinical laboratory monitoring.

Results: Changes in menopausal symptoms from baseline to end of treatment (main endpoint) were significantly superior for patients receiving *Cimicifuga racemosa* extract relative to those on placebo (p < 0.0001 for 13mg; p < 0.001 for 6.5mg). Patients' self-assessment of Quality of Life and responder rates results were similar to those for the main endpoint. Evaluation of menopausal symptoms and Quality of Life showed a high correlation (r = -0.84) between the two assessment instruments. The reported adverse events did not raise a safety concern and hormonal levels were not affected by *Cimicifuga racemosa.*

The conclusions from this clinical test can be summarized as follows: As the indication for hormone replacement gradually shifts from prevention of chronic disease to shorter-term symptom relief, *Cimicifuga racemosa* is an effective non-hormonal alternative to hormone treatment in women with menopausal symptoms.

The test results are summarized in Fig. 3 representing the correlation of Quality of Life (on the ordinate in terms of visual analogue scale) versus the Kuppermann-Index (on the abszissa).

While examples were given above, it is to be understood that these examples are for illustration and not limitation, and various changes will be apparent to those experienced in the art of preparing extracts from medical plants.

## Claims

1. A method for producing a vegetal plant extract by the steps of:
(A) treating a solid plant material with an extraction agent to obtain an extraction liquid containing said extract dissolved in said extraction agent;
(B) separating said extraction liquid from said solid plant material; and
(C) concentrating said extraction liquid by at least partially removing said extraction agent to produce a concentrated extract;
wherein said step (C) of concentrating said extraction liquid is effected in the presence of an effective amount of a pharmacologically acceptable an physiologically inert solution mediator so as to substantially avoid loss of constituents of said extract liquid upon concentration thereof by an at least partial removal of said extraction agent, said solution mediator being poly(vinylpyrrolidone).

2. The method of claim 1 wherein said extraction agent is a polar substance and preferably a C₁₋₄ alcanol or a mixture of such alcanol with water.

3. The method of claim 1 or 2 wherein said solution mediator is an organic polymer capable of being colloidally dispersible or soluble in said extraction agent.

4. The method of claim 3 wherein said organic polymer used as said solution mediator has an average molecular mass of at least about 1000 Dalton units.

5. The method of any of claims 1 - 4 wherein said vegetal plant is *Cimicifuga racemosa.*

6. An extract obtained by concentration of an extract fluid obtained by extracting of *Cimicifuga racemosa* with an extraction agent and concentration of said fluid extract in the presence of an effective amount of a solution mediator as defined in any of claims 1 - 5.

7. Use of an extract as defined in claim 6 for production of a medicament capable of binding somatic as well as central nervous receptors so as to be suitable for treating climacteric and post menopausal distress.

8. Use of an extract as defined in claim 6 for production of a medicament suitable as a substitute or complement of a hormone replacement therapy.

9. A medicament capable of substituting or complementing hormone replacement therapy, said medicament consisting at least in part of an extract obtained by concentration of an extract fluid from *Cimicifuga racemosa* in the presence of an effective amount of a PVP as a solvent mediator.

## Patentansprüche

1. Ein Verfahren zur Herstellung eines Pflanzenextraktes durch die Schritte:
(A) Behandeln eines festen Pflanzenmaterials mit einem Extraktionsmittel zur Gewinnung einer Extraktionsflüssigkeit, die den Extrakt enthält, der in dem Extraktionsmittel gelöst ist;
(B) Trennen der Extraktionsflüssigkeit von dem festen Pflanzenmaterial und
(C) Konzentrieren der Extraktionsflüssigkeit durch das wenigstens teilweise Entfernen des Extraktionsmittels zur Herstellung eines konzentrierten Extrakts;
wobei der Schritt (C) des Konzentrierens der Extraktionsflüssigkeit in der Gegenwart einer wirksamen Menge eines pharmakologisch verträglichen und physiologisch inerten Lösungsvermittlers erfolgt, um so einen wesentlichen Verlust von Bestandteilen der Extraktionsflüssigkeit beim Konzentrieren davon durch ein wenigstens teilweises Entfernen des Extraktionsmittels zu vermeiden, wobei der Lösungsvermittler Poly(vinylpyrolidon) ist.

2. Das Verfahren von Anspruch 1, wobei das Extraktionsmittel eine polare Substanz und vorzugsweise ein C₁₋₄-Alkanol oder eine Mischung solch eines Alkanols mit Wasser ist.

3. Das Verfahren von Anspruch 1 oder 2, wobei der Lösungsvermittler ein organisches Polymer ist, das in der Lage ist, in dem Extraktionsmittel kolloidal dispergierbar oder löslich zu sein.

4. Das Verfahren von Anspruch 3, wobei das organische Polymer, das als der Lösungsvermittler verwendet wird, eine mittlere Molekülmasse von wenigstens ungefähr 1.000 Dalton-Einheiten aufweist.

5. Das Verfahren von einem der Ansprüche 1 bis 4, wobei die Pflanze *Cimicifuga racemosa* ist.

6. Ein durch Konzentrieren einer Extraktionsflüssigkeit gewonnener Extrakt, der durch das Extrahieren von *Cimicifuga racemosa* mit einem Extraktionsmittel und das Konzentrieren des flüssigen Extrakts in der Gegenwart einer wirksamen Menge eines Lösungsvermittlers, wie er in einem der Ansprüche 1 bis 5 definiert wird, erhalten wird.

7. Verwendung eines Extrakts, wie er in Anspruch 6 definiert wird, zur Herstellung eines Medikaments, das in der Lage ist, somatische sowie Rezeptoren des zentralen Nervensystems zu binden, um so zur Behandlung klimakterischer und postmenopausaler Leiden geeignet zu sein.

8. Verwendung eines Extrakts, wie er in Anspruch 6 definiert wird, zur Herstellung eines Medikaments, das als ein Ersatz oder eine Ergänzung einer Hormonersatztherapie geeignet ist.

9. Ein Medikament, das in der Lage ist, eine Hormonersatztherapie zu ersetzen oder zu ergänzen, wobei das Medikament wenigstens teilweise aus einem Extrakt besteht, der durch Konzentrieren einer Extraktionsflüssigkeit aus *Cimicifuga racemosa* in der Gegenwart einer wirksamen Menge eines PVP als ein Lösungsvermittler gewonnen wird.

## Revendications

1. Procédé de production d'un extrait végétal de plante par les étapes consistant à :
A) traiter un matériau végétal solide par un agent d'extraction afin d'obtenir un liquide d'extraction contenant ledit extrait dissous dans ledit agent d'extraction;
B) séparer ledit liquide d'extraction dudit matériau végétal solide; et
C) concentrer ledit liquide d'extraction en éliminant au moins partiellement ledit agent d'extraction afin de produire un extrait concentré;
dans lequel ladite étape C) de concentration dudit liquide d'extraction est réalisée en présence d'une quantité efficace d'un médiateur en solution, pharmacologiquement admissible et physiologiquement inerte, de façon à essentiellement éviter la perte de constituants dudit liquide d'extrait lors de sa concentration par élimination au moins partielle dudit agent d'extraction, ledit médiateur en solution étant la polyvinylpyrrolidone.

2. Procédé selon la revendication 1 dans lequel l'agent d'extraction est une substance polaire et est, de préférence, un alcanol en C₁ à C₄ ou un mélange d'un tel alcanol avec de l'eau.

3. Procédé selon la revendication 1 ou 2 dans lequel ledit médiateur en solution est un polymère organique susceptible d'être dispersible de façon colloïdale ou d'être soluble dans ledit agent d'extraction.

4. Procédé selon la revendication 3 dans lequel ledit polymère organique, employé comme ledit médiateur en solution, présente une masse moléculaire moyenne d'au moins environ 1000 unités Daltons.

5. Procédé selon l'une quelconque des revendications 1 à 4 dans lequel ladite plante est *Cimicifuga racemosa*.

6. Extrait obtenu par concentration d'un liquide d'extrait obtenu par extraction de *Cimicifuga racemosa* par un agent d'extraction et par concentration dudit extrait liquide en présence d'une quantité efficace d'un médiateur en solution, comme défini dans l'une quelconque des revendications 1 à 5.

7. Utilisation d'un extrait tel que défini dans la revendication 6 pour la production d'un médicament capable de se lier à des récepteurs somatiques comme à des récepteurs du système nerveux central, de façon à être adapté au traitement d'un trouble climatérique et post-ménopausique.

8. Utilisation d'un extrait tel que défini dans la revendication 6 pour la production d'un médicament adapté comme substitut ou complément d'une hormonothérapie substitutive.

9. Médicament capable de remplacer ou de compléter une hormonothérapie substitutive, ledit médicament consistant, au moins en partie, en un extrait obtenu par concentration d'un liquide d'extrait provenant de *Cimicifuga racemosa* en présence d'une quantité efficace d'une PVP en tant que médiateur en solvant.
